(19) **Europäisches Patentamt**

**European Patent Office**

**Office européen des brevets**

(11) **EP 1 058 112 A1**

(12) # EUROPEAN PATENT APPLICATION

(43) Date of publication:
**06.12.2000 Bulletin 2000/49**

(51) Int Cl.⁷: **G01N 21/89**, G01N 33/36, B65H 63/032, B65H 63/06, G01B 11/10

(21) Application number: **99201726.9**

(22) Date of filing: **31.05.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant: **BARCO N.V.**
**8970 Poperinge (BE)**

(72) Inventor: **Zeitler, Rüdiger**
**96120 Bischberg (DE)**

(74) Representative: **Bird, William Edward et al**
**Bird Goen & Co.,**
**Vilvoordsebaan 92**
**3020 Winksele (BE)**

(54) **Improved detector and detection method for contaminants in textile materials based on an absorption measurement of light**

(57)    A detector and method for detecting contaminants in a measurement object are described where there is compensation for changes in cross-sectional area of the measurement object. Such methods and detectors are suited to detect foreign fibers in yarn slivers in a spinning mill.

The detector comprises a measurement chamber (31), preferably spherical in shape with a highly reflecting inner surface. A tube (34) allows an elongate measurement object such as a sliver to pass through the chamber (31). Absorption of light by the measurement object and/or any contaminant is measured through a transparent window (33).

Light escaping through the ends 35, 36 of tube 34 can be compensated by addition of additional light sources (41, 44) or additional photodetectors (51, 54) inside or outside the measurement chamber (31).

Fig. 9B

**Description**

The present invention relates to a detector and a detection method for contaminants such as foreign fibers or other foreign materials, in particular in yarns, slivers, ropes, unformed fiber bands on, for example (though not exclusively), carding machines, drawing benches, combing machines, flyers, open end spinning machines, winders.

**TECHNICAL BACKGROUND**

In recent years, quality conscious knitters and weavers have increasingly demanded non-contaminated yarn. Devices are known for detecting foreign materials in many stages of the process of spinning natural fibers such as cotton into yarn. It would be advantageous to be able to detect foreign fibers in slivers.

One known detector of foreign fibers suitable for the detection of foreign fibers in yarns, for example, in a spinning machine or in slivers, for example, at the output of a carding machine or on a drawbench is described in WO 98/33061 which relies upon the measurement of absorption of light within a measurement chamber. One proposed method of compensation for changes in diameter is to select a wavelength or wavelengths for the measurement light such that the sliver or yarn does not absorb a significant amount of the measurement light. One difficulty with this known device is that if the sliver or yarn has a large diameter in comparison with the measurement chamber and if there are large changes in diameter, significant and varying amounts of light may exit from the measurement chamber between the outer diameter of the sliver or yarn and the inside diameter of the openings in the measurement chamber. Further, a suitable choice of wavelengths to prevent absorption by the natural fiber may be very limited and it may only be possible to reduce the absorption coefficient of the natural fiber but not to eliminate it. In this case, there is a still a diameter dependent component in the signal which can mask foreign fiber signals or make their detection more difficult.

WO 93/19359 describes a detector for yarns or slivers in which diameter compensation is achieved by using two detectors, one sensitive to the diameter of the yarn or sliver and one for detecting the foreign fibers. Variations in the output of the second detector caused by diameter changes may be compensated for by correlation with the output of the first detector.

EP-A-884 408 describes a foreign fiber measuring device for use at the output of a carding machine which attempts to simplify the problem of foreign fiber detection by only detecting surface foreign fibers using conventional detectors.

US 5,692,267 describes an imaging apparatus for examining a fiber web before it is formed into a sliver on a carding machine and for detecting foreign materials. The use of imaging apparatus, e.g. CCD cameras, tends to make the device expensive. EP 364 786 describes a device for detecting foreign fibers in fleece before it enters the carding machine.

US 5,646,405 describes a method of detecting foreign materials in raw cotton by heating the raw cotton and detecting the infra-red signature of foreign material. The use of several cameras makes the device expensive.

There are certain disadvantages in detecting foreign materials late on in the spinning process. At the yarn stage, foreign material is preferably cut out which may cause loss of considerable lengths of yarn and machine stoppages. Hence, it would be preferable to be able to detect foreign materials reliably at an early stage. Present detectors for raw cotton and fleece achieve about 80% elimination of foreign materials. Ideally, the remaining foreign materials should be removed before the spinning operation, i.e. at the latest in the carding machine or draw bench. At this stage the fiber is in the form of a sliver, a relatively loose bundle of fibers which has already taken on the form of a rope but which does not have a precisely defined structure, e.g. no uniform diameter or consistency. Detection of foreign materials in slivers which do not have stable dimensions is difficult because many detectors are sensitive to diameter changes of the input material. These may result in false foreign fiber detection.

An object of the present invention is to provide a detector and a detection method which compensates for changes in consistency or dimensions of the textile material to be examined.

It is a further object of the present invention to provide a detector and a detection method for unformed textile materials such as slivers which is simple in operation, robust and can be manufactured economically.

**SUMMARY OF THE INVENTION**

In the rest of the description of the invention, yarns, slivers, unformed fiber bands and the like are referred to as the measurement object.

The present invention provides a method of detecting a property of a measurement object which is dependent on the cross-sectional area of the object, comprising the steps of: providing an opaque wall with an opening therein; generating a difference in light intensity across the wall so that light energy passes from one side of the wall to the other through the opening in a direction perpendicular to the wall; placing the measurement object in the opening; and measuring the change in the amount of light passing through the opening. The property to be measured may be the cross-sectional area of the measurement object. The property to be measured may also be the presence of a foreign fiber or foreign material in an elongate measurement object traveling through the opening. In this case, it is preferred if the wall defines a measurement chamber and the presence of the foreign material is determined by absorption of light in the measurement chamber by the foreign fiber. The result of measuring the light passing through the opening is used to compensate for changes in the cross-sectional area of the measurement object. The property to be measured may also be a break in an elongate measurement object traveling through the opening and the result of measuring the light passing through the opening is used to detect the break. The property may also be lack of movement of the elongate movement. This lack of movement may be detected by the fact that random variations in light intensity caused by changes in cross-sectional area of the measurement object are absent.

In a further detection method for detecting foreign fibers or foreign materials in or on a measurement object according to the present invention light which is lost from a measurement chamber by being absorbed by the measurement object in the measurement chamber is determined. From this absorption value the presence of a foreign fiber or material in or on the measurement object is determined. The method also includes a method for compensating for light lost from the chamber which has been lost by other processes than by absorption by the measurement object. Embodiments of the present invention include use of a plurality of light wavelengths or wavelength bands to compensate for light lost from the chamber which has been lost by other processes than by absorption by the measurement object. Other embodiments include the provision of light sources which allow entry of light into the measurement chamber to compensate for light lost from the chamber which has been lost by processes other than by absorption by the measurement object. Further, the present invention includes locating photodetectors so that they mainly or exclusively measure light escaping from the measurement chamber. The present invention also includes a method of continuously calibrating the level of compensation for light lost from the chamber which has been lost by other processes than by absorption by the measurement object.

The absorption of light may be measured without separate detection of reflected or transmitted light. The spectrum of the light which is used for this purpose may be selected in such a way that the measurement object does not absorb this light to a significant extent or only to a reduced extent but this is not a necessary limitation of the present invention. Use may advantageously be made of the fact that foreign fibers and foreign materials will have a greater or lesser absorption coefficient for the spectrum of the light used than the measurement object itself thus allowing detection of foreign fibers by changes in absorption of light in the measurement chamber.

The method in accordance with the present invention is preferably carried out by introducing light into the measurement chamber, in which the measurement object to be examined is situated, via at least one light source. The measurement chamber preferably substantially surrounds the measurement object. Photodetection takes place in or around this measurement chamber, with the result that all the light, or a representative sample of the light, inside the measurement chamber is measured and converted into a proportional electrical signal. To obtain a representative value of the light within the measurement chamber one or more photodetectors may be provided. the inner surface of the measurement volume is preferably coated with a reflective surface whereby a diffuse highly reflective surface is preferred. However, a mirror surfaced may also be used.

The results of a comparison of the electrical signal with:

a) a signal measured when the measurement chamber was empty; or
b) a signal measured when a representative measurement object is present in the measurement chamber; or
c) previous signals (i.e. detecting changes in the signal)

may be analyzed to provide a value representative of the loss of light from the measurement chamber of the measurement light by absorption by the measurement object, and interpretation of this absorption value may provide an indication of the presence of foreign matter in, or the level of contamination of the measurement object.

The present invention also includes a detector for detecting a property of a measurement object which is dependent on the cross-sectional area of the object, comprising: an opaque wall with an opening therein; a light source for generating a difference in light intensity across the wall so that light energy passes from one side of the wall to the other through the opening in a direction perpendicular to the wall; a measurement object located in the opening; and a photodetector for measuring the change in the amount of light passing through the opening. The property to be measured may be the cross-sectional area of the measurement object. The property to be measured may also be the presence of a foreign fiber or foreign material in an elongate measurement object traveling through the opening. In this case, it is preferred if the wall defines a measurement chamber and the presence of the foreign material is determined by absorption of light in the measurement chamber by the foreign fiber. The result of measuring the light passing through the opening is used to compensate for changes in the cross-sectional area of the measurement object. The property to be measured may also be a break in an elongate measurement object traveling through the opening and the result of measuring the light passing through the opening is used to detect the break. The property may also be lack of movement of the elongate movement. This lack of measurement object or movement thereof may be detected by the fact that random variations in light intensity caused by changes in cross-sectional area of the measurement object are absent.

A further detector according to the present invention for detecting foreign fibers or foreign materials in or on a measurement object comprises a photodetector for detecting light which is lost from a measurement chamber by being absorbed by the measurement object in the measurement chamber. Signal processing circuits can determine from this absorption value the presence of a foreign fiber or material in or on the measurement object. The detector also includes a device for compensating for light lost from the chamber which has been lost by other processes than by absorption by the measurement object. Embodiments of the present invention include light sources which emit a plurality of light wavelengths or wavelength bands and which are adapted to compensate for light lost from the chamber which has been lost by other processes than by absorption by the measurement object. Other embodiments include the provision of light sources which allow entry of light into the measurement chamber to compensate for light lost from the chamber which has been lost by processes other than by absorption by the measurement object. Further, the present invention includes locating photodetectors so that they mainly or exclusively measure light escaping from the measurement chamber. The present invention also includes a device for continuously calibrating the level of compensation for light lost from the chamber which has been lost by other processes than by absorption by the measurement object.

The present invention also includes a detector for detecting a break in an elongate object traveling through the detector or for detecting when the elongate object has stopped moving. In particular the object may be an elongate textile object and the same detector as the break or lack-of-movement detector may detect the presence of a foreign material in the elongate textile material. The elongate textile may be a rope, sliver, roving, yarn or any bundled unformed or formed textile object.

A feature of the present invention is the detection of all the light in the measurement chamber, or of a representative sample thereof which is taken uniformly over the entire measurement chamber. The detector and detection method according to the present invention does not rely for its correct functioning on the selection of a specific background or on a setting of the background illumination.

When the measurement chamber is empty, there is no absorption of light in the measurement chamber and the light which is situated in the measurement chamber, or a representative part thereof, is converted by photodetection into an electrical signal. However, light is lost through any ports into the measurement chamber which are not blocked by a measurement object.

If a non-absorbing measurement object is situated in the measurement chamber, again no light is absorbed; the light which is incident on the measurement object is returned into the measurement chamber until it is ultimately converted by photodetection into an electrical signal . This signal is different from the electrical signal with an empty measurement chamber if there are lossy ports in the measurement chamber and the measurement object blocks these ports.

However, if the measurement chamber contains a measurement object which is contaminated with a foreign fiber or foreign material which absorbs the light, not all the light incident on the measurement object is then returned into the measurement chamber. The light which is incident on the foreign fiber or the foreign material is partially absorbed thereby and the quantity of light detected in the measurement chamber is considerably less.

If the measurement chamber contains a measurement object whose absorption of the measurement light is non-negligible, the quantity of light in the measurement chamber, and thus also the measurement signal, will be dependent upon the absorption of the measurement object and therefore on its dimensions. If the volume of the measurement object in the measurement volume does not change, the measurement signal can still always be used as a measure of the contamination. Further, if light is lost from the measurement chamber by other means than absorption by the measurement object and this extra loss is dependent upon the diameter or cross-section or volume of the measurement object then even if there is no absorption of the measurement light by the measurement object, there is still a diameter, cross-section or volume dependent component to the detected signal. Embodiments of the present invention provide compensation for this dependent component.

The detector in accordance with the present invention may be installed in the creel of draw frames handling the first process drawing in a spinning mill. The position is chosen in order to detect a foreign fiber as soon as possible thus avoiding that the following steps of drawing and spinning spread the contamination over many meters of yarn. This position also allows to stop the machine immediately and to remove the fiber. A typical reaction time of the combination of the detector in accordance with the present invention and processing machinery is 50 ms which is equivalent to approximately 0.1 m of a sliver traveling at 120m/min. Other positions e.g. at the outlet of the card are also possible but the option of stopping the machine and removing the contaminant have to be taken into account.

The dependent claims define individual embodiments of the present invention. The present invention will now be described with reference to the following drawings.

## BRIEF DESCRIPTION OF THE DRAWINGS

Fig. 1 is a graph showing the effect of fractional loss and wall reflectance on the sphere multiplier of Equation 2.

Fig.2 is a graph showing the effect of wall reflectance and fractional sphere on the goodness of integration.

Fig. 3 A and B are schematic representations of a detector in accordance with an embodiment of the present invention.

Fig. 4 is the result of using spheres with different wall reflectances on thin sliver and foreign fiber detection. Top trace is for a wall reflectance of 97% and the bottom trace for a wall reflectance of about 93%. Firts peak is from a thin sliver, second peak in each trace is from a foreign fiber.

Fig. 5 is a schematic representation of a detector in accordance with another embodiment of the present invention.

Fig. 6 is a schematic representation of a detector in accordance with another embodiment of the present invention

Fig. 7 is a graph showing the substantially linear dependence of object diameter on the optical signal measured through an opening in which the object is placed.

Fig. 8 shows three traces of signals used to detect foreign fibers in accordance with the present invention. The top trace shows the trace dtected for light entering the measurement chamber. The middle trace shows signal from light detected within the measurement chamber and the bottom trace shows the sum of the two traces.

Figs. 9A to D are schematic representations of a detector in accordance with an embodiment of the present invention.

Fig. 10 is a schematic block diagram of a signal processing circuit in accordance with the present invention.

## DETAILED DESCRIPTION OF THE ILLUSTRATIVE EMBODIMENTS

The present invention will be described with reference to certain figures and embodiments but the present invention is not limited thereto but only by the claims. The present invention will be described mainly with respect to measuring foreign fibers in textiles but the invention is not limited thereto but only by the claims.

The present invention is based on the measurement of the absorption in order to detect foreign fibers on or in slivers and other textile materials, especially unformed fiber bundles, bands, ropes but also formed products such as yarns. In accordance with an embodiment of the present invention a spherical measuring chamber is used which has a significant positive effect on the signal to noise ratio (SNR), a factor 2 - 4 improvement may be obtained over a cylindrical measurement chamber. However, the present invention is not limited to a spherical measurement chamber. To measure the absorption by the measurement object in the measurement chamber, at least one light source and at least one photodetector is provided. In accordance with an embodiment of the present invention a second light source may be included in the measurement chamber emitting light with a second wavelength or second wavelength band. The amount of this second light in the measurement chamber may be used to compensate for diameter and/or cross-section changes of the measurement object, e.g. a sliver. The SNR may be increased by a factor of 5 by this technique. However, some foreign fibers absorbing IR light could not be detected. Accordingly, a preferred embodiment of the present invention includes a third light source, placed at a particular position either instead of the second light or in combination therewith.

The measurement principle of the detector according to the present invention is based on an absorption measurement within an integrated sphere (Ulbricht-Kugel) based on the theory of radiation exchange within an enclosure of diffuse surfaces. The sphere surface radiance $L_s$, which can be measured with a photodiode at an arbitrary position within the sphere, is given by

$$L_s = \frac{\Phi_i}{\pi A_s} \frac{\rho}{1-\rho(1-\Sigma f_l-\Sigma \alpha_0 f_0)} \quad \text{Equation 1}$$

where

$\Phi_i$ : flux of incident light
$A_s$ : sphere surface
$\rho$ : reflectance of sphere wall
$f_l$ : fractional loss area opening
$f_0$ : fractional area foreign fibre
$\alpha_0$ : absorption coefficient foreign fibre

As it can be seen the sphere surface radiance is dependent on the incident light and the sphere surface but also on the reflectance of the wall, the relative loss area (s) and the absorbing material, e.g. foreign fiber(s).

The second part of equation (1) is a unitless quantity which can be referred as the sphere multiplier M.

$$M = \frac{\rho}{1-\rho(1-\Sigma f_l-\Sigma \alpha_0 f_0)}. \quad \text{Equation 2}$$

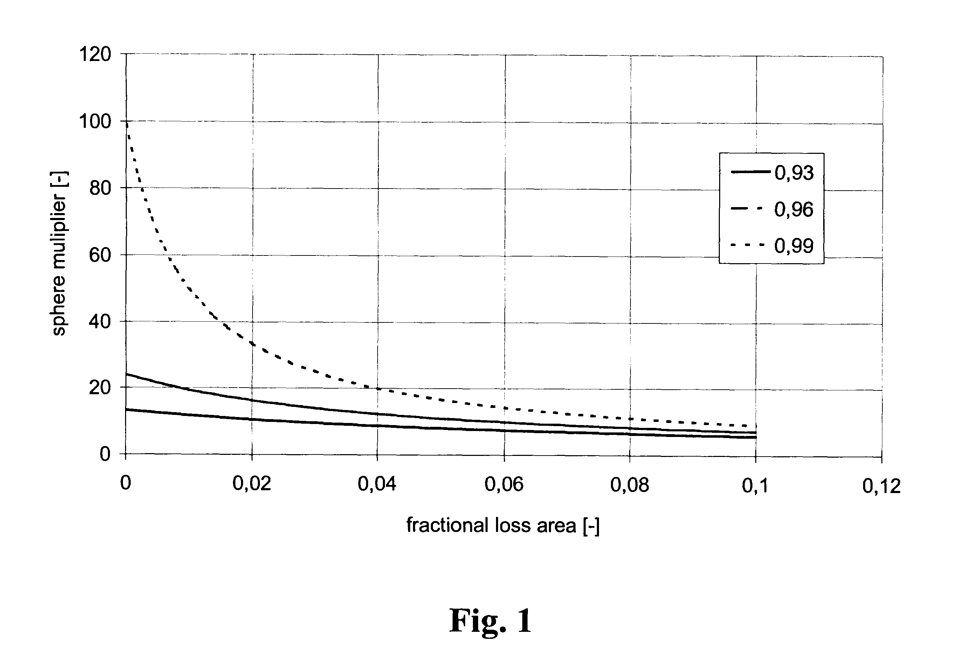

This multiplier function is shown in fig. 1 dependent on the fractional loss area and the wall reflectance. A high sensitivity is available with a high wall reflectance and with low fractional losses. In an ideal case the measured sphere surface radiance is independent of the location of the used photodetectors, e.g. photodiodes and the location of incident light. But this is only true for an ideal wall reflection ($\rho = 1$) and no losses (f= 0). The effect of the reflectance and fraction of the sphere surface lost for reflection (because an input and output port is necessary) on the spatial integration can be illustrated by considering the number of reflections required to achieve the total flux incident on the sphere surface given by equ. (1). The ratio of the flux after N reflections to the total flux is given by

$$\frac{\Phi^{\Sigma n}}{\Phi^{\Sigma \infty}} = 1 - \rho^n (1 - \Sigma f)^n. \qquad \textit{Equation 3}$$

Introducing a fixed level e.g. 95% for the fraction of the sphere surface which is contributing (some reduction must be allowed for entry and exit ports) leads to the number N of reflections required to achieve this level given by:

$$N = \frac{\ln(1 - \text{level})}{\ln(\rho(1 - \Sigma f))}, \qquad \textit{Equation 4}$$

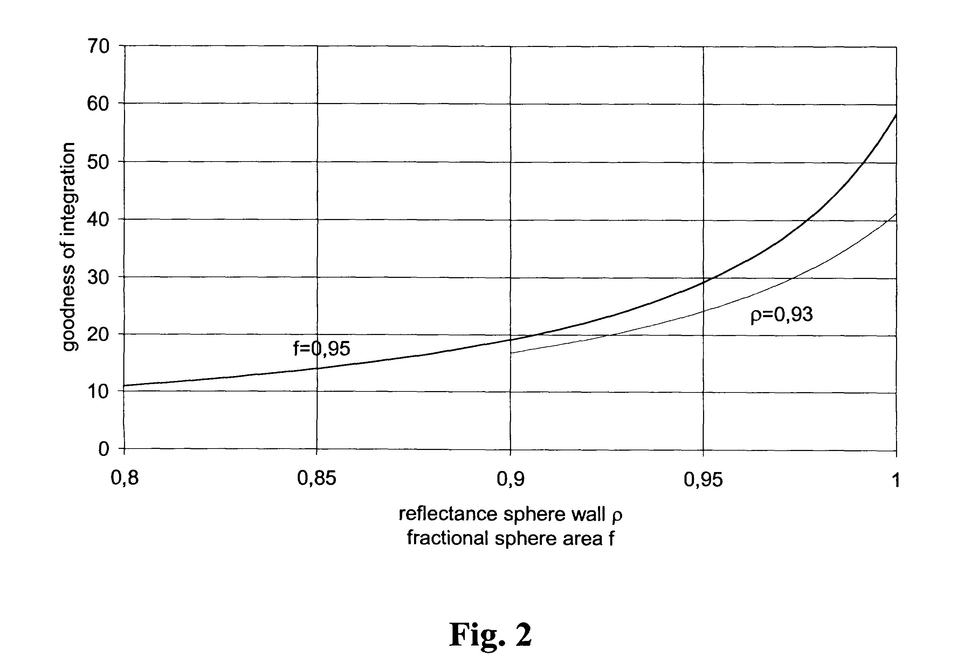

which is also a parameter of the goodness of integration. The dependencies on the wall reflectance and the fractional loss area are shown in Fig. 2. A great number of reflections (which means a good spatial integration) produces a steady state radiance as both the wall reflectance increases and the fractional losses decrease.

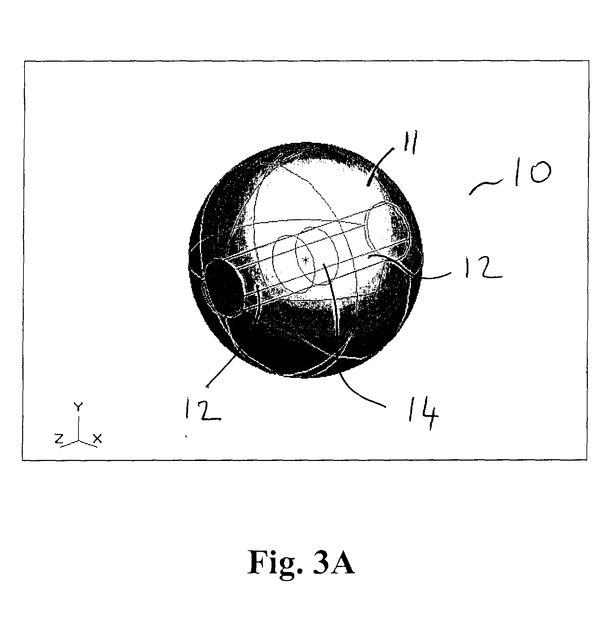

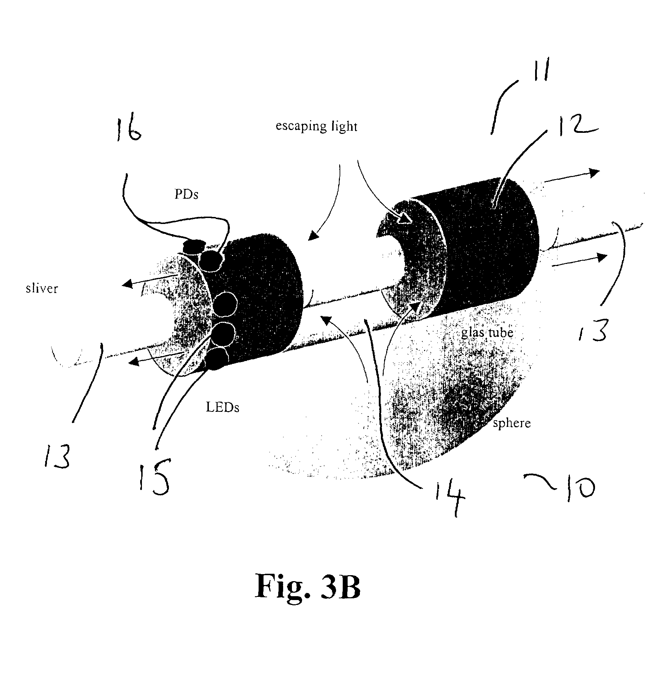

A first embodiment of the measurement chamber 10 in accordance with the present invention is shown schematically in Figs. 3A and B. It comprises a measurement chamber, for example a sphere 11. In order to detect all foreign fibers at the surface of the measurement object, for example a sliver 13, a tube 12 is mounted in the sphere 11 with the center axis of the tube 12 preferably passing through the center of the sphere 11. Tube 12 is preferably non-transparent or translucent except for a small area or window 14 in the middle thereof through which the absorption of the sliver 13 can be measured. This arrangement allows looking at the whole surface of the sliver 13 and prevents internal pollution of the sphere 11 by dust and other contaminants from the sliver 13. The sliver 13 is illuminated within the sphere 11 by means of illumination devices such as light emitting diodes (LED's) 15. The radiance or amount of light in the sphere 11 is measured with suitable photodetectors (PD's) 16.

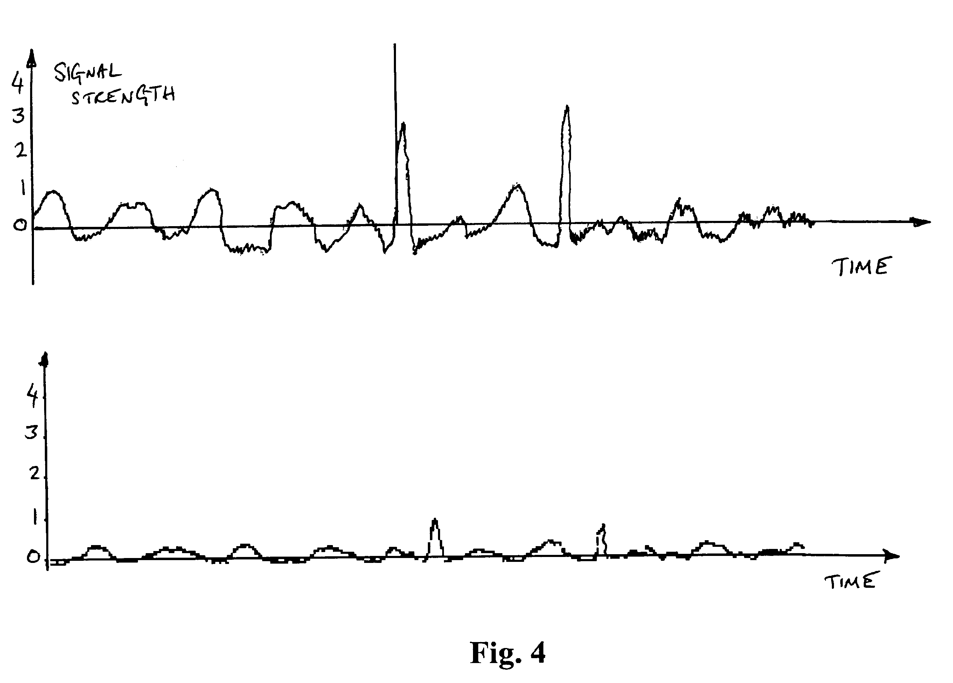

It is preferred in accordance with the present invention if the wall reflectance of the sphere 11 is greater than 90%, more preferably greater than 95%. Fig. 4 indicates the effect of wall reflectance on SNR. Two effectively identical spheres were set-up with different coatings. The first sphere was painted with a Kodak white reflectance paint 118 1759 with a reflectance of approx. 97%. The second sphere was painted with normal wall paint with a reflectance of approx. 93%. The detectors 10 were set up at the inlet of a first drawing frame one after each other. The velocity of the sliver was approx. 85m/min. In order to examine the influences of a thin sliver (e.g. a connection of two slivers) and a foreign fiber the sliver was prepared artificially. As a foreign fiber a thin piece of black wool with a length of approx. 45 mm was added. The results are shown in Fig. 4. The sphere with higher reflectance produces an output approx. 3.3 times higher than the other sphere. Based on the SNR of peak detection

$$\text{SNR}_{\text{rms}} = \frac{\text{Peak foreign fibre}}{\text{rms signal}} \qquad \textit{Equation 5}$$

the sphere with higher reflectance has an SNR 1.5 times higher than the other sphere (SNR=9.3 and SNR=6.4 respectively). The inner surface of the sphere 11 may also be metallised.

From Fig. 4, besides detection of a foreign fiber also a periodical structure can be observed in the detection signal with a period of approx. one loop of the sliver 13 in the can which carries it. The output of the detector 10 is not only dependent on the absorption of the sliver but also on its filling of the tube 12. This is also confirmed by the signal change which occurred at the thin sliver. The SNR as regards the thin sliver is given by the formula:

$$\text{SNR} = \frac{\text{peak foreign fibre}}{\text{peak thin sliver}} \qquad \textit{Equation 6}$$

and is approximately the same for both spheres (SNR=1 and SNR=0.75 respectively). For a good SNR in normal operation of the detector 10 a high wall reflectance for the sphere 11 is preferred and also the fractional area of the sphere where reflections are lost is preferably minimized. This places a limit on the ratio of the diameter of the sphere 11 and the tube 12. Preferably this ratio is chosen so that the overall loss area of the measurement chamber is less than 10 %, more preferably less than 5% of the whole inner surface area of the sphere.

Experiments have shown that the measured absorption is only slightly dependent on the sliver position in the tube 12. Preferably, the reflected light is distributed well within the sphere 11, i.e. preferably a plurality of light sources may be used, evenly distributed at least in a plane perpendicular to the direction of motion of the object to be measured. Similarly, it is preferred if a plurality of light detection devices are used which are also evenly distributed around at least a plane perpendicular to the direction of motion of the object to be measured

However, the diameter of the sliver 13 can have a pronounced effect on the amount of light lost from the measuring chamber 11. Besides the absorption of the light by the sliver 13 and/or foreign fiber a part of the light escapes through any gap between the sliver 13 and inner wall of tube 12. The amount of escaping light is dependent on the remaining opening area leading to a different radiation within the sphere 11. As the absorption of light by a foreign fiber can have the same effect on the detection signal as the loss through an opening it is preferred in accordance with an embodiment of the present invention if there is compensation for escaping light which is not lost from the measurement volume by absorption in a foreign fiber. Preferably, the compensation is carried out without reduction of SNR, in particular it is preferred if the signal to noise ratio of peak (foreign fiber) detection is increased.

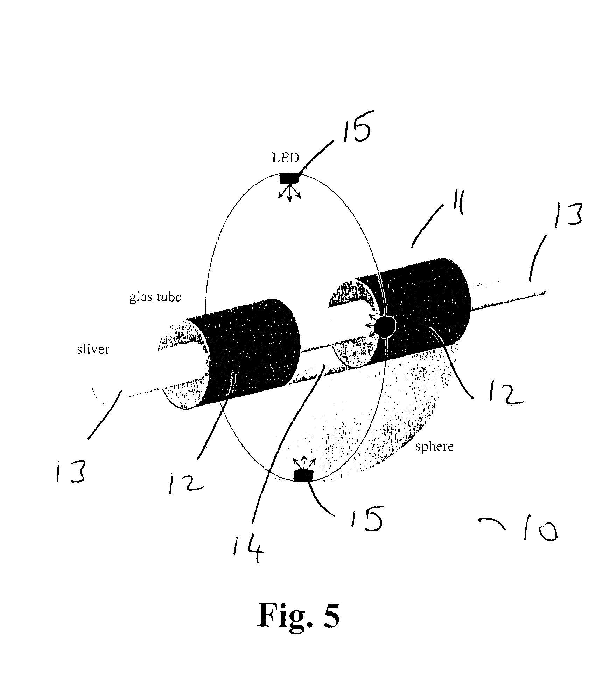

A further embodiment of the present invention is shown schematically in Fig. 5. The principle of this embodiment is to place light sources in specially selected positions to minimize light loss through the ends of the tube 12. For instance, the sliver 13 may be illuminated directly with illumination devices 15 regularly placed around a circumference of the sphere 11 and lying in a plane perpendicular to the tube 12 and centrally within the window 14. For instance, 12 SMD LED's (half-angle 60°) or 4 focused LED's (half-angle 3.5°), respectively may be arranged in the central plane on the sphere surface. In this embodiment at least the first reflection of the light is only dependent on the absorption of the sliver 13 (if any) and any foreign fiber present. This embodiment works most effectively, when the first reflection and its corresponding absorption has more energy than the light collected from the integrated sphere.

A further embodiment in accordance with the present invention for the compensation of the light escaping from the ends of tube 12 and therefore out of the measurement chamber 10 includes a second pair of light sources e.g. LED's and photodetectors which operate at another wavelength or wavelength band. An optical filter may be used to restrict the wavelengths of light incident on the second photodetectors. Preferably, this second wavelength or wavelength band is not absorbed by foreign fibers but is absorbed by the sliver 13. Hence, the photodetected signal of this second wavelength or wavelength band is related to the cross-section of the sliver 13. Calculating a difference between the signals derived from the first and second photodetectors results in an at least part elimination of the effect of the variable filling of the tube 12 by the sliver 13. With this embodiment it has been found that some diameter variations of the sliver 13 can be fully compensated and the SNR increases significantly, e.g. the SNR of peak detection may increase by a factor of 5. The compensation of a thin sliver 13, however, is still not perfect, as the system is nonlinear in respect to light losses, but the SNR as regards to a thin sliver also increases by a factor 5. As in accordance with this embodiment the difference in absorption of two wavelengths is determined, only foreign fibers with greatly different absorption coefficients at both wavelengths can be detected. A detector 10 using first LED's emitting a visible light (e.g. yellow, blue green, red or a combination) and second LED's emitting IR-light, detects most of the colored fibers likely to be encountered but some black or brown fibers which also absorb IR light may not be detected.

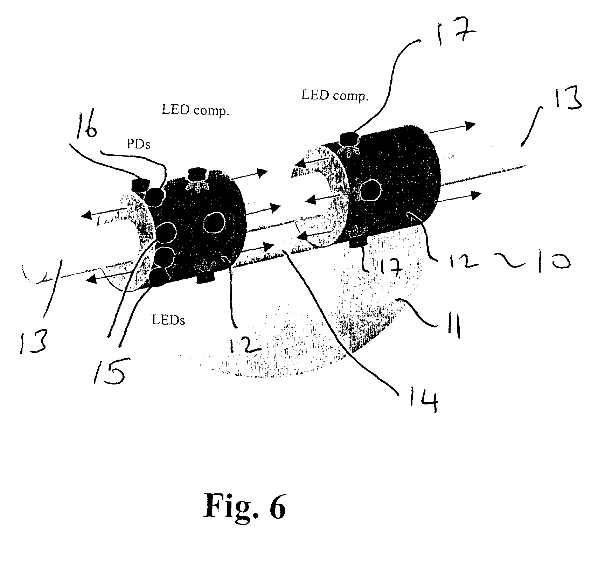

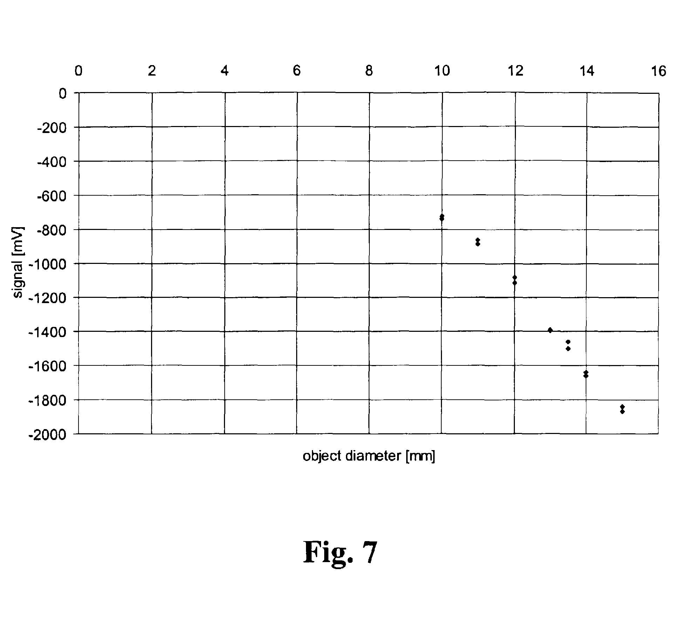

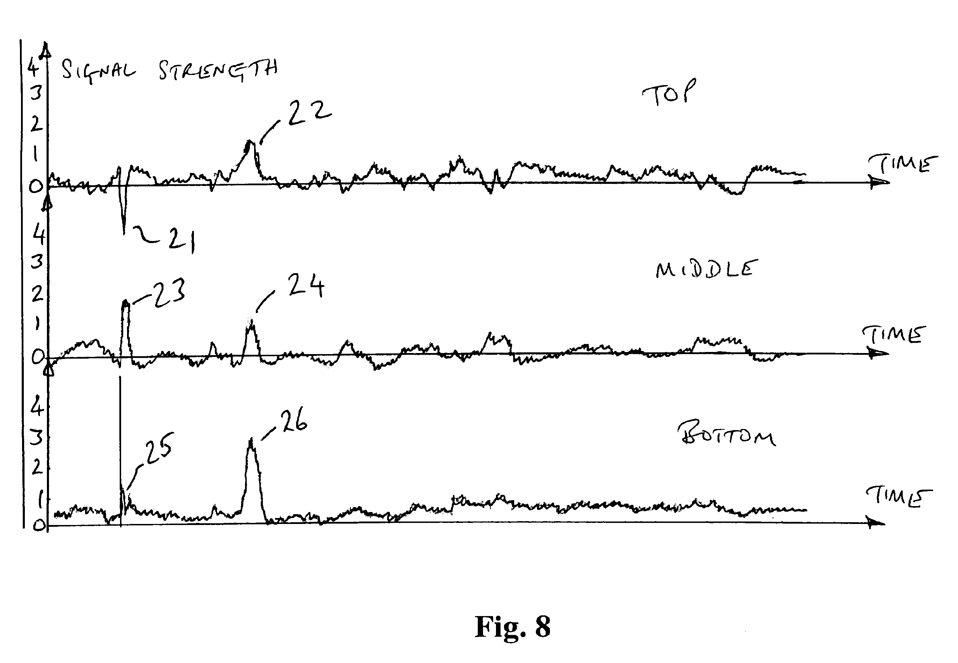

A further embodiment of the present invention is shown schematically in Fig. 6. It also relies on additional light sources but these are used to compensate for the escaping light directly (henceforth also called active compensation). No additional photodetectors are necessary but may be used. For example, a second light source 17 is placed in such a way that it shines mainly or exclusively into the tube 12 but not directly into the sphere 11. For example, LED's 17 may be placed in tube 12 so that their emitted light shines exclusively into tube 12. For instance, 2 x 3 LED's may be placed on the circumference of the tube 12 at a short distance, e.g. 15 mm from the window 14. Cylindrical objects of different diameter such as slivers where examined with only the LED's 17 for compensation emitting light. Any light which enters sphere 11 from light source 17 and is detected by photodetectors 16 does so through the gap between the sliver 13 and the inside of tube 12. The results are shown in Fig. 7. The signal from the photodetectors 16 is linearly dependent on the diameter of the object being measured with a negative slope, i.e. more light enters the sphere 11 as the diameter of the sliver 13 reduces. If the intensity of this light entering the sphere from light source 17 is adjusted to be the same as the light from light source 15 escaping from the sphere 11 under the same conditions then compensation can be obtained for changes in the diameter and shape of sliver 13. This compensation effect is shown in Fig. 8. The top trace gives the signal from photodetectors 16 when only the light source 17 is operating. At a thin portion of the sliver 13 the signal goes negative 21, i.e. more light enters the sphere 11. With a foreign fiber the signal goes positive 22 as some of the light is absorbed by the foreign fiber. The middle trace shows the output of photodetectors 16 when only light source 15 is operated. The signal goes positive 23 at a thin sliver position (more light escapes) and the foreign fiber also generates a positive going signal 24 (more light absorbed). The addition of the two signals is shown in the bottom trace in which it can be seen that the thin sliver signal 25 has been very much reduced and the foreign fiber signal 26 increased. The results obtained with this form of compensation are an SNR of peak detection increase by a factor 3.

To determine how good the compensation is the correlation between the compensating signal and the original signal can be calculated. If the light input into the measuring chamber behaves in exactly the same way as the light leaving the chamber, then the correlation should be 100%. For both the IR compensation and direct compensation method correlations in excess of 85% were obtained.

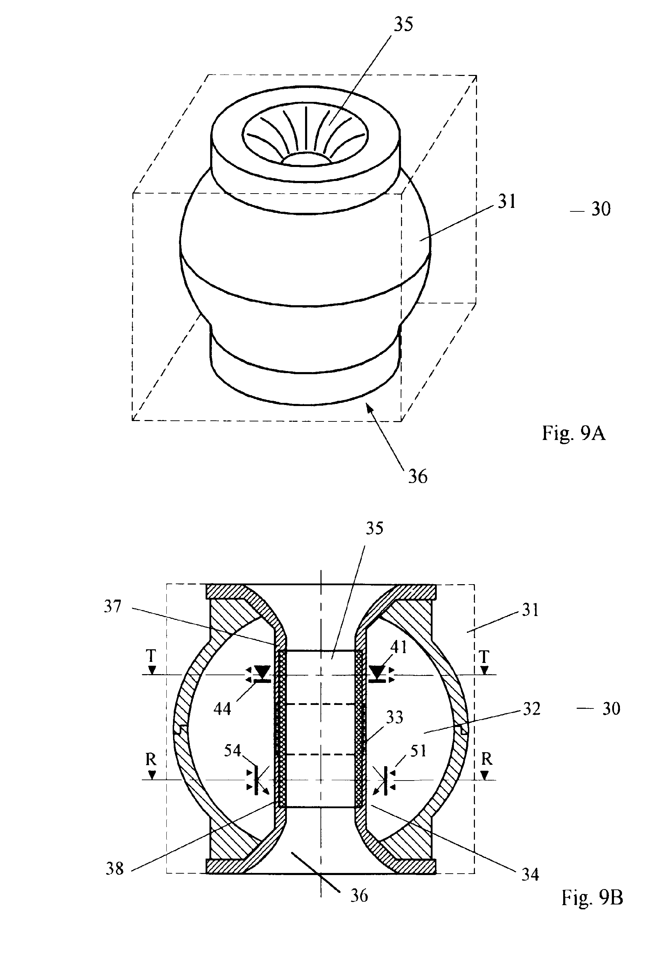

A detector 30 in accordance with a further embodiment of the present invention is shown schematically in Figs. 9A to D (best mode). Fig. 9A is a schematic representation of the outer appearance of the measurement chamber 31. Fig. 9B shows a schematic cross-sectional longitudinal view of the measurement chamber 31 (in the direction of travel of the sliver). It comprises a substantially spherical chamber 32 whose inside surface preferably has a reflectance coefficient of at least 90 %, more preferably at least 95 %. Located within the sphere 32 is a guide tube 34 having substantially cylindrical cross-section and lying within the sphere 32 as well as two flaring portions 35, 36 forming the entry and exit ports respectively. The flaring guide portions 35, 36 are preferably made from suitable hard wearing materials, e.g. ceramic, stainless steel. Within cylindrical portion 34 is located a cylindrical transparent measuring window 33 over a central region thereof, e.g. made of glass. On either side of central window 33, opaque or diffusely transparent cylindrical tubes 37, 38, e.g. made of plastic, frosted glass or transparent white ceramic, are arranged which are light and dust sealed to the inside of the spherical chamber 32. The inner surface of tubes 37, 38 merges gracefully into that of the flaring portions 35, 36, respectively. A plurality of light emitting devices 41-46, e.g. light emitting diodes, and a plurality of photodetectors 51-56 are mounted on the cylindrical portion 34. For instance, the light emitting devices 41-46 may be mounted on tube 37 and the photodetectors 51-56 may be mounted on tube 38 but the present invention is not limited thereto.

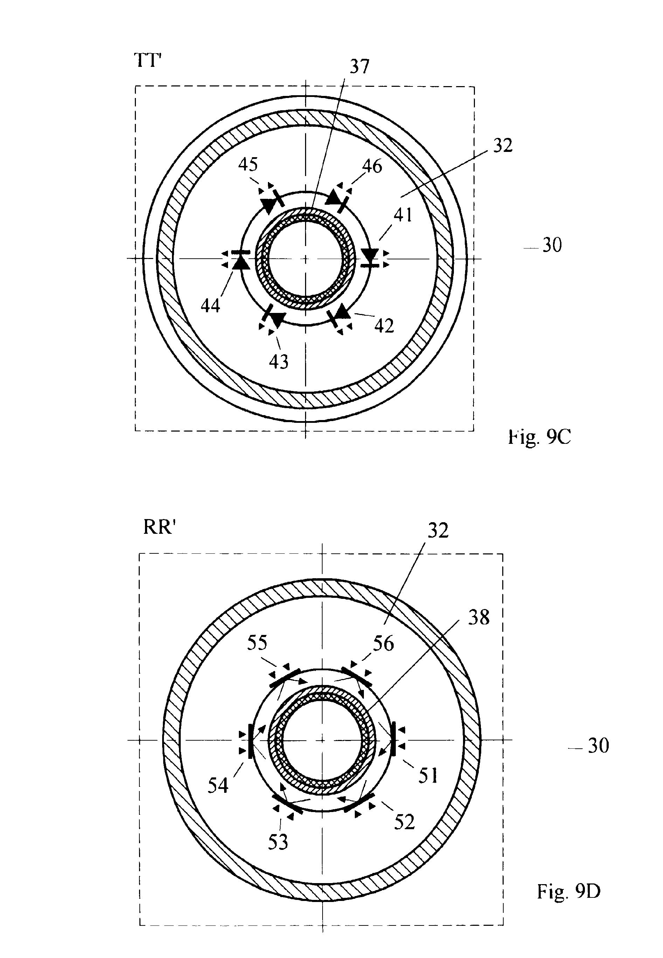

Fig. 9C is a schematic cross-sectional end view through and perpendicular to the tube 37 at the position at which the light emitting devices 41-46 are located and Fig. 9D is a schematic cross-sectional end view through the plane of tube 38 at which the photosensitive devices 51-56 are located. The detector 30 in accordance with this embodiment may be configured for:

a) wavelength-compensation. In this case, some of the photodetectors 51-56 may detect one type of light and others of the photodetectors 51-56 may detect another type of light. Additionally and optionally, some of the light emitting devices 41-46 may emit light of a different wavelength (or wavelength band) than others, e.g. may emit infra-red light. Alternatively, one type of light emitter may be used with a wide spectrum which includes two different wavelength regions, e.g. white light and infra-red light. Suitable filters may be placed on or integrated with either or both of the light emitting devices 41-46 and the photodetectors 51-56 to provide two or more wavelength operation or two or more wavelength band operation. The purpose of using a second wavelength for detection is to select the second wavelength so that it is not absorbed by the foreign fibers significantly but reacts to changes in dimensions of the sliver in the same way as the main detection signal. This second signal may then be used to compensate or cancel out variations in the main detect signal caused by the changes in cross-sectional area of the sliver.

b) direct or active compensation. In one embodiment of the present invention some of the light emitted from the emitting devices 41-46 is arranged to shine into the tube 37 and some of the light shines into the spherical chamber 32. This can be achieved by making the tube 37 and/or the tube 38 transparent. However, it is preferred for calibration reasons if the ratio of light energy which shines into the spherical chamber 32 and the light energy which enters the tube 37 and/or tube 38 is variable and settable. Hence, the present invention includes a means for settably varying the ratio of the light energy which shines into the spherical chamber 32 and the light energy which enters the tube 37 and/or tube 38. During calibration this ratio can be set so that the light escaping through tubes 37, 38 between the measurement tube 34 and the sliver can be compensated for by light entering the sphere through the gap between the tube 37 and/or tube 38. Note that as shown in Fig. 9, the light emitting devices 41-46 for shining mainly or exclusively into tube 37 and/or 38 are mounted inside the spherical chamber 32. The present invention is not limited thereto and includes these light sources being mounted outside the spherical chamber 32 and shining into tube 37 and/or tube 38.

In another embodiment of the present invention the photodetectors are partitioned between sphere and tube and not the light emitters. In this embodiment some of the photodetectors are arranged to measure mainly or exclusively the light in the tube 37 or 38 and some of the photodetectors are arranged to measure mainly the light in the sphere 32. There is no need to have separate light sources but these may be added as well. By directly measuring the light in the tube 37, 38 a measure of the light escaping from the sphere 32 is obtained. This may be used to compensate for the light leaving the sphere, i.e. may be used to compensate for the changes in cross-sectional area of the sliver. The photodetectors for measuring the light escaping through the tube 37 or 38 may be located outside the sphere 32.

c) a combination of wavelength diameter compensation and direct diameter compensation. In this case some of the photodetectors 51-56 may detect one wavelength or wavelength band, and other devices 51-56 may detect another one or more wavelengths or wavelength bands, e.g. infra-red as described above under a). Also the detector 30 may include a means for settably varying the ratio of light energy which shines into the spherical chamber 32 and the light which enters the tube 37 and/or tube 38 as described above under b).

The photodetectors 51-56 sense the amount of light of a particular wavelength or wavelength band present in the chamber 32 and convert this into an electrical signal. Foreign material in the sliver absorbs a fraction of the light emitted by the light emitting devices 41-46 and this changes the electrical signal from the photodetectors 51-56. Light is absorbed from all angles, i.e. substantially from all over the spherical chamber 32, thus allowing an uninterrupted view of the contamination, regardless of its position in the sliver. Preferably, the shape of the guide tube 34 is such that no sliver compression takes place. A preferred embodiment of the present invention includes:

**[0053]** - Three different light sources 41-46. As the whole amount of light is collected with only one set of receivers 51-56, each light source may have the same spectrum but be amplitude or frequency modulated in order to separate the channels afterwards. By modulation of the measurement light at a specific frequency, it is also possible to distinguish it from ambient light and thus to suppress the latter effectively as well as to distinguish it from other light sources in the measurement chamber.

The following channels may be provided:

- Channel 1: Light source using a combination of yellow and green shining into the spherical chamber 32. A change in cross-section of the sliver as well as the absorption of a foreign fiber influences this channel.
- Channel 2: Light source using another wavelength or wavelength band, e.g. IR-LED's shining into the sphere 32 in order to apply an IR-compensation. The wavelength is chosen such that most of the dyes used for the fibers of the sliver do not absorb in this spectral range. A change in cross-section of the sliver is mainly measured by monitoring this light.
- Channel 3: Light source using yellow LED's shining into the tube 37, 38 in order to apply an active (direct) diameter compensation. The wavelength of the LED's is similar to the first light source. A changing cross-section of the sliver as well as the absorption of a foreign fiber influences this channel. The electrical signal caused by a change in sliver cross-section is in the opposite direction from that from channel 1. This inverse signal change is used to cancel (compensate) diameter influence on channel 1.

The present invention also includes a signal processing circuit for processing the signals from photodetectors 51-56. For example, the following electrical output signals may be provided:

- a linear combination of channel 1-3 to eliminate changing sliver cross section and detect foreign fiber with simple peak detection.
- a filtering (e.g. correlation) of channel 1, 2, 3 in order to eliminate the effects of changing sliver cross-section and emphasize foreign fiber signal.
- channel 2 output to measure the cross-sectional area or cross-sectional variations of a sliver.
- channel 3 output to detect thin connections of two slivers.
- a filtered value of channel 1, 2 or 3 to detect sliver movement or sliver break.

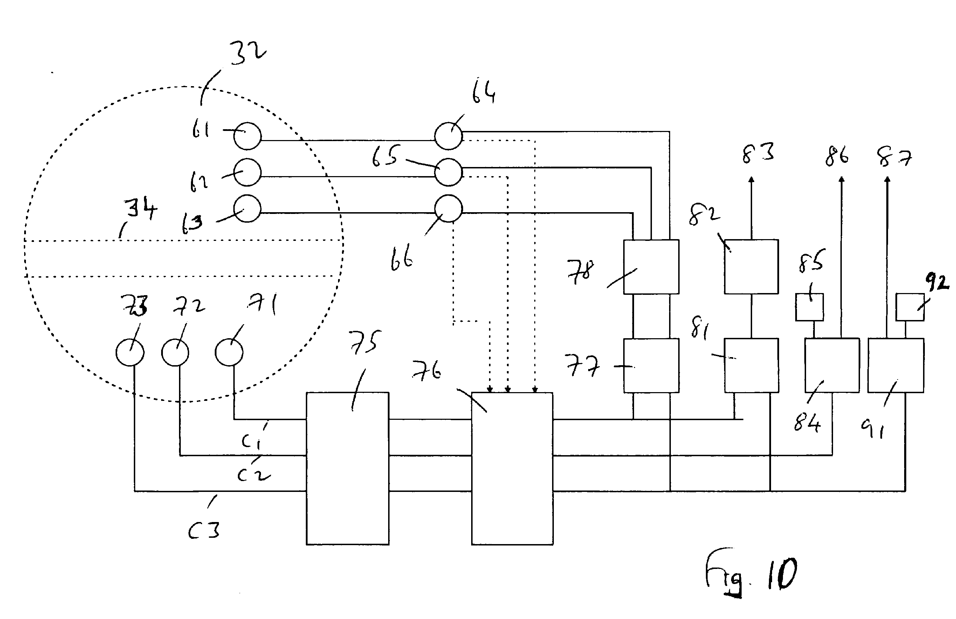

A signal processing unit 60 in accordance an embodiment of the present invention is shown in Fig. 10. Three light emitting devices 61, 62, 63 or sets of light emitting devices may be provided for emitting light into the measurement chamber 32 and/or the measurement tube 34 for the three channels C1 to C3 mentioned above. Each emitting device 61-63 is driven by a suitable frequency or amplitude modulated power source, 64, 65, 66, respectively. Further, a set of photodetectors 71-73 may be provided for receiving light from the measurement volume 32. The outputs from the photodetectors 71-73 may be processed in a signal pre-processor 75 which may include suitable pre-amplifiers and band-pass filters to exclude other forms of noise, e.g. electromagnetic including radio interference and 50Hz mains hum. The output from the pre-processor 75 is then fed to an extraction circuit 76 for extraction of the signals from channels C1-C3. The extraction circuit 76 may be designed depending upon the method of modulation of the light sources. For instance, it may include bandwidth filters, or signals from sources 64-66 may be multiplied with the signal received from the pre-processor 75 in order to extract the signals from the three channels C1-C3.

A linear combination of the signals from channels 1 and 3 may be derived in an adding means 81. The output from the adding means 81 is delivered to a discriminating engine 82 which detects peaks generated by foreign fibers. Once a foreign fiber is detected a signal 83 may be generated which may be used to sound an alarm or to stop the sliver processing machine.

The output from channel 2 may be fed to a comparator 84 in which the magnitude of this signal is compared with a set value from set value circuit 85. The comparator 84 outputs a signal 86 related to the cross-sectional area of the sliver which may be used for quality control purposes or may be used to detect a sliver break (no cross--sectional area). A sliver break signal may be used to stop the sliver processing machine.

The output from channel 3 may be fed to a comparator 91 in which the magnitude of this signal is compared with a set value from circuit 92 for the detection of sliver thin spots.

In accordance with a further embodiment of the present invention a means is provided for continuous calibration of the compensating effect of the light which enters the tube 37, 38 for compensation purposes. One way of doing this is to calculate the magnitude of the standard deviation of the signals from channels 1 and 3 above separately. Normally, (no foreign fiber present) variations in the output of channels 1 and 3 are caused by the variations in sliver cross-section, e.g., shape and/or diameter. As these variations have the same source, their magnitude should be made the same if the compensation is perfect. If the absolute magnitudes are the same the magnitudes of the standard deviations should also be the same or closely correlated. Hence, an additional component in the signal processing may be a processing engine to calculate the standard deviation (e.g. least squares) of the signals from channels 1 and 3, to compare these and to adjust the light intensities of the light emitting devices for channel 1 and 3 to provide an exact compensation of the light entering and leaving the spherical chamber 32. This continuous calibration function is provided without interrupting the detection activity of detector 30. For instance with reference to Fig. 10 the signals of channels C1 and C3 may fed to a processing engine 77 such as a microprocessor for individually calculating the standard deviations of the signals from channels C1 and C3 over a predetermined period (running standard deviation determination). The standard deviations of the signals in these two channels C1 and C3 are then compared in a comparator 78 and a difference signal derived which is used to set the relative power levels delivered by power sources 64 and 66 so that there is exact compensation of the light lost from the measurement chamber 32 and the compensation light entering the chamber. The value of the standard deviations may be used to determine if there is a break in the sliver or if it has stopped moving. Under these circumstances the signal becomes almost constant so the the standard deviations reduce by up to orders of magnitude. This abrupt change in noise levels on the received signals may be used to indentify a break in the sliver or if the sliver has stopped moving (break downstream of the detector). The signal can be sued to start an alarm or to stop the processing machine.

With the signal-processing unit it is sufficient to activate the light source 41-46 using the correct electric current (for example using DC for an LED), and to amplify the detected photoelectric signal to a usable level using a suitable amplifier.

The absolute value of the light detected can be used to derive an absolute measure of the absorption of the light employed by the measurement object. In another approach, which takes account only of the variation of the light detected, the relative absorption can be derived by comparison with an uncontaminated measurement object.

By evaluating the level of the electrical signal, it is possible to establish whether a foreign fiber or foreign material is present in the measurement chamber and hence in the measurement object.

This evaluation can lead to assigning a contamination index in an automated manner to the measurement object, by associating a contamination index with each absorption.

While the invention has been shown and described with reference to preferred embodiments, it will be understood by those skilled in the art that various changes or modifications in form and detail may be made without departing from the scope and spirit of this invention as defined in the attached claims. For example, the electrical signals from the photodetectors 41-46 can be processed further in a conventional manner by means of analog or digital circuits or using programmable digital signal processing or microprocessor circuits. It is thus possible to take into account the amplitude and the length of the deviation, for example in order to decide whether it is necessary to interrupt the movement of the measurement object or the processing which the measurement object is undergoing, in order to remove the deviating parts from the measurement object, or for statistical characterization of the measurement object. Processing and using the signals in this way is usual both for detectors of foreign fibers which are based on other principles and for measurement equipment based on diameter or mass measurements which is known from the prior art.

The spectrum of the measurement light has a considerable influence on the types of foreign fibers and foreign materials which can be detected using the detector according to the invention. This is linked to the spectral absorption properties of these contaminating materials. Some foreign fibers and foreign materials in the measurement object can be perceived visually as a change in intensity or a discoloration. Extensive tests have demonstrated that this visible contamination can best be detected using yellow or green measurement light.

Other foreign fibers and foreign materials cannot be distinguished visually from the measurement object, but are of a different type of material. A frequently occurring contamination of cotton is, for example, polypropylene. Both look virtually white in their natural form. However, it is possible to distinguish the two materials from one another both in ultraviolet and in near infrared by their different specific absorption of this light. By selecting the appropriate wavelength or band of wavelengths of the measurement light, it is possible to detect foreign fibers and foreign materials on the basis of the type of material instead of on the basis of their color. The wavelengths at which the types of material differ in absorption are typically situated in the near infrared between 1000 nm and 5000 nm.

The light source used in the present invention does not necessarily have to be a light-emitting diode, also known as LED, but may also be an incandescent lamp or an arc lamp which fills the measurement chamber with light, either directly or indirectly, via one or more optical conductors. As an alternative light source, it is possible to use, for example, electroluminescent materials. A layer of a material of this kind is applied to a substrate. This substrate may take the form of a cylinder which adjoins or forms tube 37 or 38. In order to make the light uniform over the measurement chamber, the light source is preferably disposed in such a way that the space inside the measurement chamber is illuminated uniformly.

One or more photosensitive elements can be used for the photodetection. Their number is not a limitation on the present invention, but it is preferably that there is an arrangement which allows all the light in the measurement chamber, or a representative sample thereof, to be measured. The photosensitive elements used must be sufficiently sensitive to the spectrum of the measurement light emitted by the light source.

The photodetectors may, for example, be silicon photodiodes, but the invention is not limited to such detectors. These photodiodes are suitable for the detection of light in the visible spectrum and the near infrared (typically 400 to 1100 nm). Other photodetectors are known to the person skilled in the art for both shorter wavelengths (UV) and for longer wavelengths (IR), such as for example germanium or InGaAs photodiodes.

Photodiodes are mostly constructed as discrete components. The current state of technology also allows photodetectors to be applied to a substrate in the form of an active layer. It is conceivable that this substrate may adopt a form which adjoins the tube 37 or 38 or forms the tube 37 or 38.

If one color or wavelength or band of wavelengths is used, it may be that a number of different contaminating materials of the measurement object will be difficult or impossible to detect. For this reason, as an extension to the present invention it is possible to use illumination which comprises a plurality of colors or wavelengths or bands of wavelengths. The combination can be optimized for the detection of specific contaminants.

To this end, it is possible to use the light from one or more broad-band light sources, such as for example an arc lamp or incandescent lamp, the spectral distribution of which is optionally adapted to the requirements by active or passive optical filtering.

As an alternative, it is possible to use a combination of light sources, different light sources or groups of light sources each emitting light of a different color or wavelength or band of wavelengths.

With illumination of this kind, the detection can be carried out by measuring the total resulting quantity of light in the measurement chamber, or a representative, uniform sample thereof This is used to obtain a total weighted absorption for the colors or wavelengths or bands of wavelengths used.

As an alternative, the absorption can be measured separately for the various colors or wavelengths or bands of wavelengths. In order to separate the signals detected for these various colors or wavelengths or bands of wavelengths from one another, the light sources or groups of light sources which emit a different color or wavelength or band of wavelengths can be switched on and off sequentially or can be intensity-modulated at a different frequency. The signals can be separated from one another using synchronous detection or electronic filtering.

Separation into different colors or wavelengths or bands of wavelengths is also possible by means of active or passive optical filters which are placed in front of one or more photosensitive elements.

In the above the detection of foreign fibres has mainly be described. But the methods and detectors mentioned above are able to detect a wide variety of contaminants such as seeds, dirt, dusts, oil and other color stains.

## Claims

1. A method for detecting a contaminant, for example foreign fibres or foreign materials in or on an elongate measurement object travelling through a measurement chamber, in which the amount of light lost from the measurement chamber by being absorbed by the measurement object is measured and the presence of a foreign fibre or material in or on the measurement object is determined from this value, the method further comprising the step of: compensating for escaping light from the chamber which has been lost through changes in the cross-sectional area of the measurement object.

2. The method according to claim 1, further comprising use of a plurality of light wavelengths or wavelength bands to compensate for effect of the escaping light on the detection.

3. The method according to claim 1, further comprising the provision of light sources which allow entry of light into the measurement chamber to compensate for the effect of escaping light on the detection.

4. The method according to claim 1, further comprising the provision of at least one photodetector for measuring the escaping light.

5. The method according to any of claims 1 to 4, further comprising continuously calibrating the level of compensation for the escaping light.

6. The method according to any of claims 1 to 5, further comprising the step of detecting a break in, or lack of movement of the measurement object.

7. A detector for detecting a contaminant for example foreign fibers or foreign materials in or on a measurement object comprising a photodetector for detecting light which is lost from a measurement chamber by being absorbed by the measurement object in the measurement chamber; a signal processor for determining from this absorption value the presence of a foreign fiber or material; and a device for compensating for light escaping from the chamber caused by changes in cross-sectional area of the measurement object.

8. The detector according to claim 7, wherein the device for compensation includes light sources which emit a plurality of light wavelengths or wavelength bands.

9. The detector according to claim 7 or 8, wherein the compensation device includes a light source which allows entry of light into the measurement chamber.

10. The detector according to any of claims 7 to 9, wherein the compensation device includes locating a photodetector so that it mainly or exclusively measures light escaping from the measurement chamber.

11. The detector according to any of claims 7 to 10, further comprising a device for continuously calibrating the level of compensation for light lost from the measurement chamber.

12. The detector according to any of the claims 7 to 11, further comprising a device for detecting a break in or a lack of motion of the elongate measurement object.

13. The detector according to any of the claims 7 to 12, wherein the inner surface of the measurement chamber has a reflectance of at least 90%.

14. The detector according to any of claims 7 to 13, wherein the measurement chamber is a sphere.

15. The detector according to claim 14, further comprising a tubular member within the sphere, the tubular member having a transparent portion substantially in the middle thereof.

16. A method of detecting a property of a measurement object which is dependent on the cross-sectional area of the object, comprising the steps of:

providing an opaque wall with an opening therein;
generating a difference in light intensity across the wall so that light energy passes from one side of the wall to the other through the opening;
placing the measurement object in the opening; and measuring the change in the amount of light passing through the opening.

17. The method according to claim 16, wherein the property to be measured is the cross-sectional area of the measurement object.

18. The method according to claim 16, wherein the property to be measured is the presence of a contaminant in an elongate measurement object travelling through the opening.

19. The method according to claim 18, wherein the wall defines a measurement chamber and the presence of the contaminant is determined by absorption of light in the measurement chamber by the contaminant and the result of measuring the light passing through the opening is used to compensate for changes in the cross-sectional area of the measurement object.

20. The method according to claim 16, wherein the property to be measured is a break in an elongate measurement object travelling through the opening and the result of measuring the light passing through the opening is used to detect the break.

21. The method according to claim 16, wherein the property to be measured is the lack of movement of an elongate measurement object travelling through the opening and the result of measuring the light passing through the opening is used to detect the break.

22. A detector for detecting a property of a measurement object which is dependent on the cross-sectional area of the object, comprising: an opaque wall with an opening therein; a light source for generating a difference in light intensity across the wall so that light energy passes from one side of the wall to the other through the opening in a direction perpendicular to the wall; a measurement object located in the opening; and a photodetector for measuring the change in the amount of light passing through the opening.

23. The detector according to claim 22, wherein the wall defines a measurement chamber and the measurement object is an elongate measurement object traveling through the opening, the property to be measured is the presence of contaminant for example a foreign fiber or foreign material in or on the measurement object, further comprising: a device for detecting the presence of the contaminant by determining the absorption of light in the measurement chamber by the contaminant.

24. The detector according to claim 23, further comprising a device for compensating for changes in the cross-sectional area of the measurement object.

25. A detector or a method according to any previous claim wherein the measurement object is a textile material, for example a roving, a sliver, a yarn, a rope, or a thread.

**Fig. 1**

EP 1 058 112 A1

**Fig. 2**

EP 1 058 112 A1

**Fig. 3A**

**Fig. 3B**

**Fig. 4**

EP 1 058 112 A1

LED

15

11

13

glas tube

sliver

13

12

14

12

sphere

15

~10

**Fig. 5**

**Fig. 6**

**Fig. 7**

**Fig. 8**

EP 1 058 112 A1

Fig. 9A

Fig. 9B

TT'

Fig. 9C

RR'

Fig. 9D

EP 1 058 112 A1

Fig. 10

**European Patent Office**

**EUROPEAN SEARCH REPORT**

Application Number

EP 99 20 1726

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 5 371 584 A (SCHEINHUETTE HANS-JUERGEN) 6 December 1994 (1994-12-06)<br><br>* abstract *<br>* column 2, line 55 - line 66 *<br>* figures 1-3 *<br>--- | 1,3,5,7, 9,11,13, 17,24,25 | G01N21/89<br>G01N33/36<br>B65H63/032<br>B65H63/06<br>G01B11/10 |
| D,A | WO 98 33061 A (BARCO NV ;BOUVYN PATRICK (BE)) 30 July 1998 (1998-07-30)<br>* abstract *<br>* page 10, line 34 - page 11, line 5 *<br>* page 12, line 17 - line 35 *<br>* figures 1-3 *<br>--- | 1,2,7,8 | |
| A | US 4 645 922 A (WELBOURN CHRISTOPHER M ET AL) 24 February 1987 (1987-02-24)<br><br><br>* abstract *<br>* column 1, line 27 - line 43 *<br>* column 1, line 59 - column 2, line 5 *<br>* column 5, line 18 - line 51 *<br>* figure 1 *<br>--- | 1,2,7,8, 13,14, 16,17, 22-24 | |
| A | US 4 663 522 A (WELBOURN CHRISTOPHER M ET AL) 5 May 1987 (1987-05-05)<br>* abstract *<br>* column 1, line 37 - line 41 *<br>* column 3, line 6 - line 12 *<br>* figure 2 *<br>--- | 1,7,13, 14,16,22 | TECHNICAL FIELDS SEARCHED (Int.Cl.7)<br><br>G01N<br>G01B<br>B65H |
| A | EP 0 652 432 A (BARCO AUTOMATION NV ;SCHLAFHORST & CO W (DE)) 10 May 1995 (1995-05-10)<br>* abstract *<br>* figure 3 *<br>--- | 1,7,25 | |
| | | -/-- | |

The present search report has been drawn up for all claims

| Place of search | Date of completion of the search | Examiner |
|---|---|---|
| THE HAGUE | 20 October 1999 | Verdoodt, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

European Patent
Office

**EUROPEAN SEARCH REPORT**

Application Number

EP 99 20 1726

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 5 383 017 A (SCHUERCH GEORG) 17 January 1995 (1995-01-17) * abstract * * column 1, line 51 - line 60 * * figure 1 * | 16-19, 22,23,25 | |
| A | US 4 341 958 A (OHSAWA SHIUJIA) 27 July 1982 (1982-07-27) * abstract * * figure 3 * | 6,12,20 | |

TECHNICAL FIELDS
SEARCHED     (Int.Cl.7)

The present search report has been drawn up for all claims

| Place of search | Date of completion of the search | Examiner |
|---|---|---|
| THE HAGUE | 20 October 1999 | Verdoodt, E |

EPO FORM 1503 03.82 (P04C01)

**ANNEX TO THE EUROPEAN SEARCH REPORT
ON EUROPEAN PATENT APPLICATION NO.**

EP 99 20 1726

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-1999

| Patent document cited in search report | | Publication date | Patent family member(s) | | Publication date |
|---|---|---|---|---|---|
| US 5371584 | A | 06-12-1994 | CH | 683294 A | 15-02-1994 |
| | | | DE | 59209665 D | 29-04-1999 |
| | | | EP | 0553445 A | 04-08-1993 |
| | | | JP | 5264469 A | 12-10-1993 |
| WO 9833061 | A | 30-07-1998 | BE | 1010882 A | 02-02-1999 |
| | | | AU | 5742898 A | 18-08-1998 |
| US 4645922 | A | 24-02-1987 | AU | 599240 B | 12-07-1990 |
| | | | AU | 1770188 A | 22-09-1988 |
| | | | AU | 584922 B | 08-06-1989 |
| | | | AU | 4835785 A | 10-04-1986 |
| | | | BE | 903379 A | 03-02-1986 |
| | | | CA | 1276809 A | 27-11-1990 |
| | | | GB | 2165644 A,B | 16-04-1986 |
| | | | GB | 2198551 A,B | 15-06-1988 |
| | | | IN | 166216 A | 31-03-1990 |
| | | | IN | 168359 A | 16-03-1991 |
| US 4663522 | A | 05-05-1987 | AU | 584923 B | 08-06-1989 |
| | | | AU | 4835885 A | 10-04-1986 |
| | | | BE | 903378 A | 03-02-1986 |
| | | | CA | 1257781 A | 25-07-1989 |
| | | | GB | 2165943 A,B | 23-04-1986 |
| | | | IN | 166215 A | 31-03-1990 |
| EP 0652432 | A | 10-05-1995 | JP | 7325049 A | 12-12-1995 |
| US 5383017 | A | 17-01-1995 | CH | 683035 A | 31-12-1993 |
| | | | DE | 59209666 D | 29-04-1999 |
| | | | EP | 0553446 A | 04-08-1993 |
| | | | JP | 5273152 A | 22-10-1993 |
| US 4341958 | A | 27-07-1982 | GB | 2092187 A,B | 11-08-1982 |

EPO FORM P0459

For more details about this annex : see Official Journal of the European Patent Office, No. 12/82